(19) Europäisches Patentamt
European Patent Office
Office européen des brevets

(11) Publication number: 0 164 623
B1

(12) **EUROPEAN PATENT SPECIFICATION**

(45) Date of publication of patent specification: 30.08.89

(51) Int. Cl.[4]: A 61 B 1/00

(21) Application number: 85106294.3

(22) Date of filing: 22.05.85

(54) **Fiberscope with bending mechanism.**

(30) Priority: 30.05.84 JP 112123/84

(43) Date of publication of application:
18.12.85 Bulletin 85/51

(45) Publication of the grant of the patent:
30.08.89 Bulletin 89/35

(84) Designated Contracting States:
DE FR GB IT NL SE

(56) References cited:
DE-B-1 102 341
GB-A-1 086 821
US-A-3 572 325
US-A-3 583 393
US-A-3 960 143

(73) Proprietor: SUMITOMO ELECTRIC INDUSTRIES LIMITED
No. 15, Kitahama 5-chome Higashi-ku
Osaka-shi Osaka 541 (JP)

(72) Inventor: Tsuno, Koichi c/o Osaka Works
Sumitomo Electric Ind. Ltd. No.1-3 Shimaya
1-chome
Konohana-ku Osaka-shi Osaka (JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 164 623 B1

Courier Press, Leamington Spa, England.

**Description**

The invention relates to a fibrescope according to the generic clause of claim 1.

Most fiberscopes using an image fiber interalia gastroscopes require frequent change in the direction of the imaging section by external manipulation.

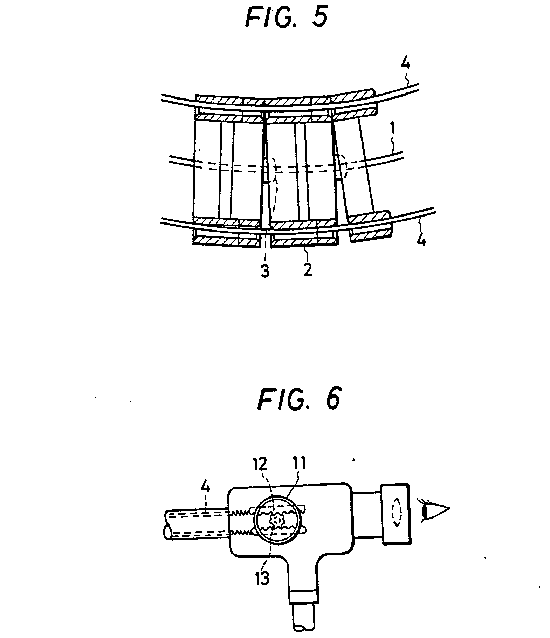

A conventional mechanism for meeting this requirement is illustrated by Fig. 5. As seen in said drawing an image fiber is surrounded by a plurality of node rings 2 which are caused to abut against each other with an intervening central spherical fulcrum 3. Manipulating wires 4 are inserted through the ring nodes 2. The manipulating wires are controlled by the mechanism shown in Fig. 6 so as to permit free bending of the image fiber 1.

A fiberscope having the features of the precharacterizing clause of claim 1 is disclosed in the US—A—3583393. This fiberscope uses a bendable tube assembly comprised of a plurality of thin walled cylindrical segments arranged in series endwise to each other. The end edge of the segments is bevelled starting from diametrically opposed two points in the end edge so that two diametrically opposed raised portions are formed in the end edge thereby permitting the series of the segments connected endwise to each other to be bent.

The formation of the single segments however, leads to a complex constructional design and further to a fiberscope having a relatively large thickness that results from the use of the node rings and segments respectively.

The object underlying the present invention is therefore to fabricate a very fine fiberscope having an easy construction particularly for bending mechanism.

To solve this task the fiberscope according to the generic clause of claim 1 is characterized by the features of the characterizing clause of said claim.

Preferred embodiments of the invention are disclosed in the depending claims.

The invention will now be described with reference to the drawings.

Fig. 1 shows one embodiment of the mechanism for realizing the bending of the tip of the fibrescope in accordance with the present invention.

Fig. 2 shows an example of the construction of the tip of the same fiberscope.

Fig. 3 shows an example of the tip casted section of the same fiberscope.

Fig. 4 illustrates a manipulating wire sheathed inner tube in coil form used in the fiberscope of the present invention;

Fig. 5 illustrates the conventional bending mechanism; and

Fig. 6 shows the manipulation wire control mechanism at the image receiving section of the same fiberscope.

Description of the preferred embodiments

A preferred embodiment of a fiberscope of the present invention is shown in Figs. 1 to 4. This fiberscope is especially designed for viewing the inner walls of the heart. In order to ensure a wide field of view and eliminate any opaque blood, a transparent balloon 5 is provided at the forward end of the fiberscope as shown in Fig. 1. As depicted in Fig. 3, the internal components of the scope are an image fiber 1, light-guiding plastic fiber bundles 18, and balloon expanding or contracting tubes 6. At the tip of the image fiber 1 is provided a sleeve 8 which is connected to an imaging lens 9. In order to ensure easy bending of the fiber, two manipulating wires 4 sheathed in tubes in a coil form 7 are provided diametrically in the cross section of the fiber. The tubes 7 are made of a material such as stainless steel that ensures good slip on the manipulating wires 4.

The device shown in Fig. 1 (which does not show the image fiber and light guide), has an assembly of elements 17 (including the imaging lens parts of the image fiber the light guide and the balloon expanding or contracting tubes 6 and other tip-forming elements) that are cast and fixed in an epoxy resin or other suitable materials, thereby providing a tip cast section 10. The tubes 7 in a coil form extend halfway through an intermediate cast section 14 and only the manipulating wires 4 extend to the tip cast section 10. As shown in Figs. 3 and 4, the tip of each manipulating wire 4 is folded back and fixed within the tip cast section. In the embodiment shown in Fig. 1, the intermediate cast section 14 is spaced from the tip cast section and has the image fiber and the light guide cast and fixed therein. The image fiber and the light guide is held free between the two cast portions. That part of the image fiber which is between these two cast sections is covered with a rubber tube 15 made of silicone rubber or other material that satisfy the requirements: (1) sufficient resiliency to provide an adequate resistance against the bending forces exerted by manipulation with the wires 4, and (2) sufficient resiliency to provide an adequate resistance against radial expansion of the image fiber, the light guide and said tube 6. With the construction described above, the outer diameter of the cable section of the fiberscope in accordance with the present invention can be reduced to a dimension as small as 2.8 mm. The manipulating wires 4 have outside and inside diameters of approximately 0.53 mm and 0.19 mm, respectively. The bending mechanism incorporated in the scope of the present invention permits free bending through ±45° under a tension of about 1.4 kg.

Fig. 2 shows the construction of the forward portion of the fiberscope in accordance with the present invention, and Fig. 3 illustrates the construction of the tip cast section 10.

In order to provide greater slip on the manipulating wires, the inner surfaces of the tubes 7 in coil form may be coated with a polyethylene tetrafluoride resin such as Teflon R. Bending in more than two directions may be achieved by using a greater number of manipulating wires.

As described in the foregoing, the fiberscope of the present invention replaces the conventionally used node rings by a flexible rubber tube that provides an outer cover for bending section and through which the manipulating wires are inserted. The bending mechanism incorporated in the fiberscope does not result in an increase in diameter; instead, this mechanism contributes to a reduction in the cost and weight of the bending portion.

**Claims**

1. A fiberscope comprising a cover tube (16), an image fiber (1) and light guides (18) for transmitting light within said cover tube, a tip portion including the end of the light guides and said image fiber to illuminate and to image a region to be observed, said tip portion being spaced to the forward end of said cover tube (16), said image fiber and light guides extending through said forward end of said cover tube up to the tip portion, and with a bending mechanism for flexibly connecting the tip portion with the cover tube, said bending mechanism comprising manipulating wires extending through said cover tube up to the tip portion and being fixed to the tip portion, said wire being guided through a tube (7) in a coil form, said tube (7) ending in the region of the forward end of said cover tube (16), characterised in that said bending mechanism comprises an intermediate cylindrical cast section (14) inserted into the forward end of said cover tube (16), protruding out of said forward end and having the form of a moulded portion in which the image fiber (1), light guides (18) and said tubes (6, 7) are fixed, said bending mechanism further comprising a tip cast section (10) forming said tip portion, said tip cast section (10) being in the form of a moulded portion, in which the light guides (18), tubes (6) and wires (4) are fixed, said tip cast section (10) and said intermediate section (14) being flexibly connected by a resilient rubber tube (15), having sufficient resiliency to provide an adequate resistance against the bending forces exerted by manipulation with the wires (4) and having sufficient resiliency to provide an adequare resistance against radial extension of the image fiber and the light guide.

2. The fiberscope according to claim 1, wherein said tube (7) having an inner surface provided with a coating of polyethylene tetrafluoride resin.

3. A fiberscope according to claim 1 or 2, wherein the tip of each of said manipulating wires (4) is folded back and fixed in the tip casted section (10).

4. The fiberscope according to claim 1, wherein said rubber tube (15) comprises silicone rubber.

5. The fiberscope in accordance with any one of the foregoing claims, wherein said manipulating wires (4) are provided diametrically opposite in the cross-section of said fiberscope.

6. The fiberscope according to any one of the foregoing claims, further comprising a transparent balloon (5) disposed at the forward end of the tip casting section (10).

**Patentansprüche**

1. Endoskop mit einem Hüllrohr (16), einer Abbildungsfaser (1) und Lichtleitern (18) zur Übertragung von Licht innerhalb des genannten Hüllrohrs, einem das Ende der Lichtleiter und der genannten Abbildungsfaser enthaltenden Endabschnitt zur Beleuchtung und Abbildung eines zu beobachtenden Bereichs, wobei der genannte Endabschnitt vom vorderen Ende des genannten Hüllrohrs (16) beabstandet ist und die genannte Abbildungsfaser und die Lichtleiter sich durch das genannte vordere Ende des genannten Hüllrohrs bis zum Endabschnitt erstrecken, und mit einem Biegemechanismus zum flexiblen Verbinden des Endabschnitts mit dem Hüllrohr, wobei der genannte Biegemechanismus Handhabungsdrähte umfaßt, die sich durch das genannte Hüllrohr biz zum Endabschnitt erstrecken und am Endabschnitt befestigt sind, der genannten Draht durch ein Rohr (7) in Spiralform hindurchgeführt ist und das genannte Rohr (7) im Bereich des vorderen Endes des genannten Hüllrohrs (16) endet, dadurch gekennzeichnet, daß der genannte Biegemechanismus einen mittleren zylindrischen Gußabschnitt (14) umfaßt, der in das vordere Ende des genannten Hüllrohrs (16) eingesetzt ist, aus dem genannten vorderen Ende herausragt und die Form eines in einer Form hergestellten Teils aufweist, in dem die Abbildungsfaser (1), die Lichtleiter (18) und die genannten Rohre (6, 7) befestigt sind, der genannte Biegemechanismus ferner einen den genannten Endabschnitt bildenden End-Gußabschnitt (1) umfaßt, wobei der genannte End-Gubabschnitt (10) die Form eines in einer Form hergestellten Teils aufweist, in welchem die Lichtleiter (18), die Rohre (6) und die Drähte (4) befestigt sind, und der genannte End-Gußabschnitt (10) und der genannte mittlere Abschnitt (14) mittels eines elastischen Gummirohrs bewegbar verbunden sind, welches ausreichende Elastizität aufweist, um einen angemessenen Widerstand gegenüber den bei der Handhabung mittels der Drähte (4) ausgeübten Biegkräften zu erzeugen, und ausreichende Elastizität aufweist, um einen angemessenen Widerstand gegenüber radialer Vergrößerung der Abbildungsfaser und des Lichtleiters zu erzeugen.

2. Das Endoskop nach Anspruch 1, in welchem das genannte Rohr (7) eine Innenfläche aufweist, die mit einer Beschichtung aus Polyäthylen-Tetrafluoridharz versehen ist.

3. Ein Endoskop nach Anspruch 1 oder 2, in welchem das Ende jedes der genannten Handhabungsdrähte (4) zurückgefaltet und im End-Gußabschnitt (10) befestigt ist.

4. Das Endoskop nach Anspruch 1, in welchem das genannte Gummirohr (15) Silikongummi umfaßt.

5. Das Endoskop nach einem der vorhergehenden Ansprüche, in welchem die genannten Handhabungsdrähte (4) im Querschnitt des genannten Endoskop diametral gegenüberliegend vorgesehen sind.

6. Das Endoskop nach einem der vorhergehenden Ansprüche, ferner mit einem durchsichtigen Ballon (5), der am vorderen Ende des End-Gußabschnitts (10) angeordnet ist.

**Revendications**

1. Endoscope comprenant un tube de gainage (16), une fibre image (1) et des guides de lumière (18) destinés à transmettre la lumière à l'intérieur dudit tube de gainage (16), une portion de pointe comprenant l'extrémité de guides de lumière et de ladite fibre image et destinée à éclairer et à former l'image d'une région à observer, ladite portion de pointe étant espacée de l'extrémité avant du tube de gainage, ladite fibre image et lesdits guides de lumière traversant ladite extrémité avant dudit tube de gainage, jusqu'à la portion de pointe, et un mécanisme de courbure destiné à relier la portion de pointe au tube de gainage par une liaison flexible, ledit mécanisme de courbure comprenant des fils de manipulation qui s'étendent dans ledit tube de gainage jusqu'à la portion de pointe et qui sont fixés à la portion de pointe, ledit fil étant guidé dans un tube enroulé (7), ledit tube (7) se terminant dans la région de l'extrémité avant dudit tube de gainage (16), caractérisé en ce que ledit mécanisme de courbure comprend une section coulée cylindrique intermédiaire (14) insérée dans l'extrémité avant dudit tube de gainage (16), qui émerge en dehors de ladite extrémité avant et revêt la forme d'une portion moulée dans laquelle la fibre image (1), les guides de lumière (18) et lesdits tubes (6, 7) sont fixés, ledit mécanisme de courbure comprenant en outre une section de pointe coulée (10) qui forme ladite portion de pointe, ladite section de pointe coulée (10) étant constituée par une portion moulée dans laquelle les guides de lumière (18), les tubes (6) et les fils (4) sont fixés, ladite section de pointe coulée (10) et ladite section intermédiaire (14) étant reliées selon une liaison flexible, par un tube en caoutchouc élastique (15), qui possède une résistance vive suffisante pour opposer une résistance appropriée aux forces de courbure exercées par la manipulation au moyen des fils (4) et ayant une résistance vive suffisante pour opposer une résistance appropriée à la dilatation radiale de la fibre image et du guide de lumière.

2 Endoscope selon la revendication 1, dans lequel ledit tube (7) possède une surface interne munie d'un revêtement de résine de tétrafluorue de polyéthylène.

3. Endoscope selon la revendication 1 ou 2, dans lequel la pointe de chacun desdits fils de manipulation (4) est repliée en arrière et fixée dans la section de pointe coulée (10).

4. Endoscope selon la revendication 1, dans lequel ledit tube de caoutchouc (15) est constitué par du caoutchouc de silicone.

5. Endoscope selon une quelconque des revendications précédentes, dans lequel lesdits fils de manipulation (4) sont prévus dans des positions diamétralement opposées dans la section transversale dudit endoscope.

6. Endoscope selon une quelconque des revendications précédentes, comprenant en outre un ballonnet transparent (5) disposé à l'extrémité avant de la section de pointe coulée (10).

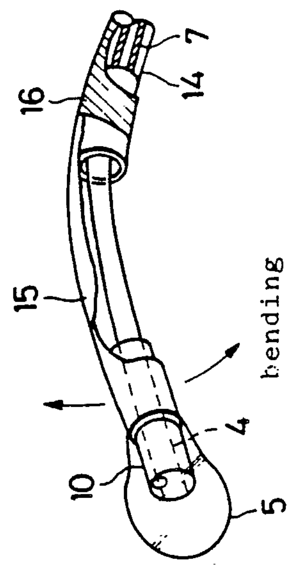


FIG. 1

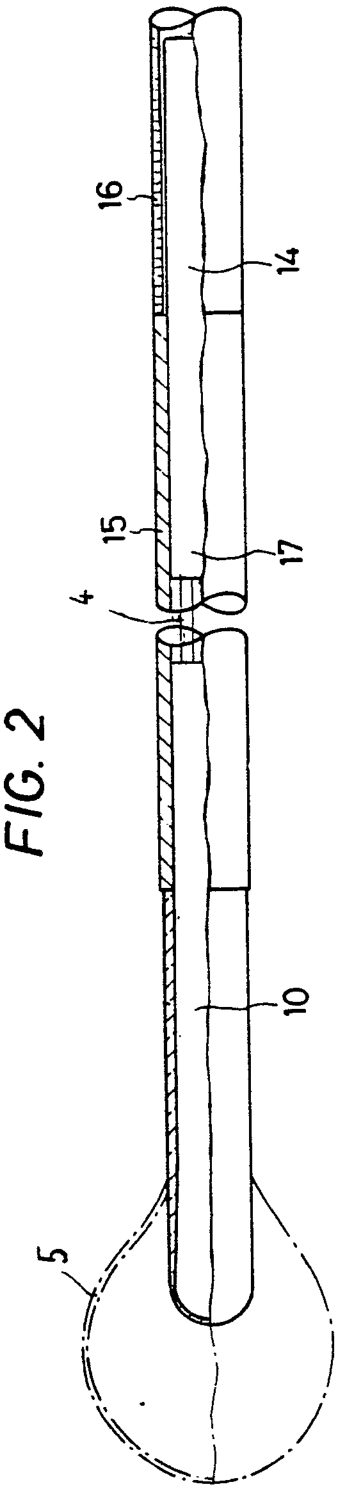


FIG. 2

FIG. 3

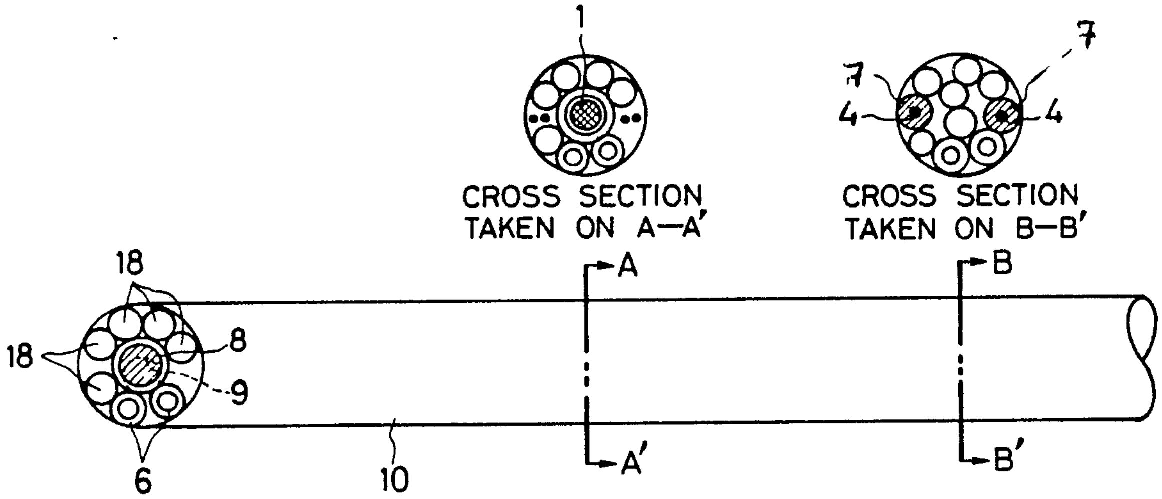
1
7
7
4
4
CROSS SECTION
TAKEN ON A—A'
CROSS SECTION
TAKEN ON B—B'
18
A
B
18
8
9
6
10
A'
B'

FIG. 4

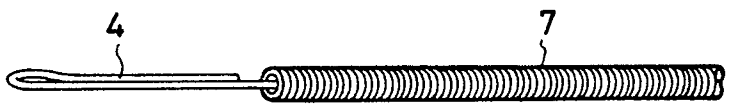
4
7

FIG. 5

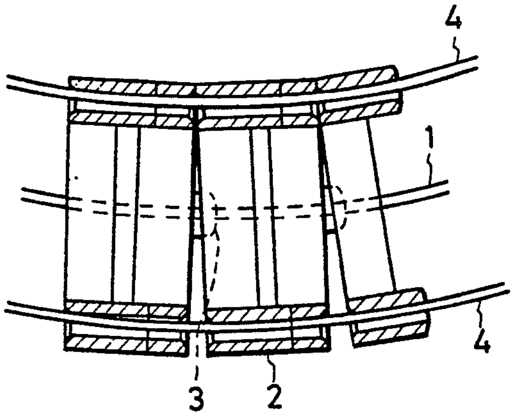


FIG. 6